# EUROPEAN PATENT SPECIFICATION

(11) **EP 1 450 860 B1**
(45) Date of publication and mention of the grant of the patent: **18.09.2013**
(21) Application number: 02776186.5
(22) Date of filing: 07.10.2002
(51) Int. Cl.: A61K 47/00, A23K 1/165, A23K 1/17

(54) **ANIMAL FEEDS INCLUDING ACTIVES**
TIERFUTTER MIT WIRKSTOFFEN
ALIMENTS POUR ANIMAUX COMPRENANT DES PRINCIPES ACTIFS

(30) Priority: 05.10.2001 US 971521; 05.10.2001 US 971518; 05.10.2001 US 971519; 05.10.2001 US 971520
(43) Date of publication of application: 01.09.2004
(73) Proprietor: Rubicon Scientific LLC, Sabetha, KS 66534 (US)
(72) Inventor: HUBER, Gordon, R., Sabetha, KS 66534 (US); JONES, David, R., Palm Beach, FL 33480 (US); KUENZI, John, C., Bern, KS 66048 (US); KUENZI, Kevin, D., Perry, KS 66073 (US); CABRERA, Francisco, A., Overland Park, KS 66212 (US)
(74) Representative: UEXKÜLL & STOLBERG
(86) International application number: PCT/US2002/032125
(87) International publication number: WO 2003/030653

(56) References cited:
- EP-A2- 0 231 825
- WO-A-02/00202
- WO-A1-99/17625
- US-A- 3 357 884
- US-A- 4 199 569
- US-A- 4 597 969
- US-A- 5 456 933
- US-A- 5 589 503
- US-A- 5 750 548
- US-A- 5 952 033
- US-A- 6 117 477
- DATABASE WPI Section Ch, Week 198017 Derwent Publications Ltd., London, GB; Class B02, AN 1980-30071C XP002338129 -& JP 55 035055 A (OISHI I) 11 March 1980 (1980-03-11)
- B. GJERDE: "Eradication of Otodectes cynotis infection in farmed blue and silver foxes (Alopex lagopus and Vulpes vulpes) with ivermectin in the feed" NORSK VETERINAER TIDSSKIRFT, vol. 108, no. 2, 1996, pages 75-81, XP008050168 NONORSKE VETERINRFORENING, OSLO
- DATABASE WPI Week 198733, Derwent Publications Ltd., London, GB; AN 1987-231555 & JP 62 155051 A (HOECHST AG) 10 July 1987

## Description

### BACKGROUND OF THE INVENTION

### Field of the Invention

The present invention is broadly concerned with improved daily ration feed products for animals including minor amounts of an active or drug such as a pharmaceutical drug. More particularly, the invention is concerned with such feed products, and methods of preparing and using the products, wherein the feeds contain a sufficient quantity of an active or drug such as a heartworm preventative drug so that when the animals consume the feeds, therapeutically effective amounts of the active or drug are established and maintained in the bloodstreams of the animals. In this way, conventional dosing regimes are eliminated, and the animals receive proper quantities of the active as a part of their normal daily diets.

### Description of the Prior Art

In recent years there has been a significant increase in animal research directed to determining proper nutritional standards and also effective drug treatments for animals. This is true not only in connection with domestic household pets such as dogs, cats, birds, and exotics, but also in regard to economically significant animals such as farm animals (e.g., horses, sheep and cattle) and zoo animals of all types.

Drug or active treatment of animals generally requires that these agents be administered from time to time by oral administration or injection, so that therapeutic amounts of the actives or drugs can be maintained in the bloodstreams of the animals either continuously or at least during a prescribed treatment period. Periodic dosing presents a number of difficulties. For example, the animal's caretaker may simply forget to administer a given drug or active at the required time. This can have the effect of disrupting a treatment protocol and even causing harm to the animal. For instance, dogs are conventionally treated with heartworm preventative drugs such as ivermectin on a monthly basis. If the dog's owner forgets to timely administer the drug, the dog is susceptible to heartworm infection. Another problem associated with periodic dosing of animals stems from the fact that the animals may be very reluctant to cooperate, especially if the drug or active is to be orally administered. Any cat owner can testify to the difficulty of persuading a domestic cat to consume a drug product.

A large number of actives can be used in the context of the invention, so long as the actives can withstand feed processing conditions and retain their potency. The actives are antibiotics, steroids, anti-inflammatory agents, endoectacides (e.g., dewormers such as heartworm-preventative drugs) and ectoparasiticides (e.g., drugs effective against fleas and ticks).

Heartworm infection is an endemic condition with certain animals, and especially household pets such as cats and dogs. A number of actives or drugs have been developed for the treatment of heartworm infection, such as the avermectins, which are a class of macrocyclic lactones. Drugs of this class include ivermectin, selamectin, moxidectin, milbemycin oxine and eprinomectin.

Ivermectin is a known oral and injectable medication used as a wormer, heartworm preventative and to kill certain mites (mange). Ivermectin is a mixture of (10*E*,14*E*,16*E*,22*Z*)-(1*R*,4*S*,5'*S*,6*S*,6'*R*,8*R*,12*S*,13*S*,20*R*,21*R*,24*S*)-6'-[(*S*)-*sec*-butyl]-21,24-dihydroxy-5',11,13,22-tetramethyl-2-oxo-(3,7,19-trioxatetracyclo[15.6.1.1^{4,8}.0^{20,24}]pentacosa-10,14,16,22-tetraene)-6-spiro-2'-(perhydropyran)-12-yl 2,6-dideoxy-4-*O*-(2,6-dideoxy-3-*O*-methyl-αa-L-*arabino-*hexopyranosyl)-3-*O*-methyl-αa-L-*arabino*-hexopyranoside and (10*E*,14*E*,16*E*,22*Z*)-(1*R*,4*S*,5'*S*,6*S*,6'*R*,8*R*,12*S*,13*S*,20*R*,21*R*,24*S*)-21,24-dihydroxy-6 '-isopropyl-5 ',11,13,22-tetramethyl-2-oxo-(3,7,19-trioxatetracyclo[15.6.1.1^{4,8}.0^{20,24}]pentacosa-10,14,16,22-tetraene)-6-spiro-2'-(perhydropyran)-12-yl 2,6-dideoxy-4-*O*-(2,6-dideoxy-3-*O*-methyl-αa-L-*arabino-*hexopyranosyl)-3-*O*-methyl-αa-L-*arabino*-hexopyranoside CAS: #70288-86-7.

Selamectin is identified as (5*Z*,25*S*)-25-cyclohexyl-4'-*O*-de(2,6-dideoxy-3-*O*-methyl-αa-L-*arabino*-hexopyranosyl)-5-demethoxy-25-de(1-methylpropyl)-22, 23-dihydro-5-(hydroxyimino)avermectin A₁ₐ.

Moxidectin is SPIRO[11,15-METHANO-2H,13H,17H-FURO[4,3,2-PQ][2,6]-B ENZODIOXACYCLO-OCTADECIN-13,2'[2H]PYRAN-17-ONE]-6'-[1,3-DIMETHYL-1-BUTENYL]-3',4',5',6,6',7,10,11,14,15, 17a,20,20a,20b-DIHYDRO-4'-[METHOXYIMINO]-5',6,6,19-TETRAMETHYL-[6R-2aE,4E,4'E,5'S*,6R*,6'S*(E),8E, 11R*, 13R*, 15S*, 17aR*, 20R*, 20aR*, 20bS*]].

Milbemycin Oxime consists of the oxime derivatives of 5-didehydromilbemycins in the ratio of approximately 80% A4 (C32H45N07, MW 555.71) and 20% A3 (C31H43N07).

Eprinomectin is 4"-epiacetylamino-4"-deoxyavermectin B₁.

These drugs are conventionally provided in tablet form or, for larger animals, as pastes and injectable liquids. Generally, animals are treated with relatively large doses of these drugs on a periodic basis. In the case of dogs and cats, tablets/chewables are given once a month by mouth year round for heartworm prevention. Higher doses are used to eliminate other parasites.

I vermectin is the most commonly used heartworm preventative drug in domestic pets, and is generally considered safe at recommended dosage levels. If these are exceeded, side effects such as tremors, staggering, dilated pupils, loss of body weight or death may occur. As a consequence of normal dosing regimes for ivermectin, the treated animals necessarily receive a relatively large quantity of the drug which is to remain effective for an extended period. This in turn means that shortly after treatment the animal has a very high concentration of ivermectin in its bloodstream, with this concentration tailing off during the remainder of the period. This is to be contrasted with a more preferable treatment protocol wherein a substantially constant level of ivermectin is maintained on a continuing basis.

By the same token, the other established heartworm preventative drugs are generally administered in the same fashion as ivermectin, i.e., a relatively large quantity of the drugs are given at intervals, rather than daily administration of the drug to achieve a maintenance level in the animal's bloodstream.

Attempts have been made in the past to provide daily ration products which include therapeutic drugs. For example, Hills Pet Food Products made and sold a Science Diet product referred to as "Maximum Stress Diet" which included small amounts of styrylpyridinium chloride and diethylcarbamazine in a canned dog food containing substantial quantities of animal fat which required refrigeration. However, the Maximum Stress Diet is no longer available, and was not optimum in that it required refrigeration and special handling. This is to be contrasted with conventional extruded feed products designed to be stored over extended periods at ambient temperature without significant loss of nutrients.

U.S. Patent No. 6,190,591 describes a single-extruder process for the production of controlled release particles which may be tableted. Various encapsulants includingpharmaceuticals, nutraceuticals, nutritional compounds, biologically active components, flavorants, fragrances, detergents and surface-active compositions are described, at relatively large quantities in the particles of at least 1% and preferably from about 3-50%. Hence, the '591 patent is not concerned with complete feeds, but rather encapsulant particles. The process described in this patent makes use of an elongated extruder where water and lipid are successively injected into the barrel, followed by water evaporation from the barrel and final addition of encapsulants. Such equipment is generally not suited to the production of a daily ration feed or similar product, given the need to uniformly distribute an active in the latter type of product.

U.S. Patent No. 5,550,153 describes methods for killing adult heartworms in dogs by the administration of ivermectin or similar drugs. The '153 patent teaches that such drugs may be incorporated into a canine feed ration. Nevertheless, the contemplated treatment regime is on a monthly basis, i.e., one dosage of ivermectin is given to the animal each month. Accordingly, this patent does not address the provision of a daily ration feed and the amount of ivermectin present in the once per month feeds is very high. Hence, the treatment regime according to this patent still suffers from the problem of delivering a very high concentration of drug immediately upon dosing, with a continual falloff of drug in the animal's bloodstream thereafter.

JP 62155051 WPI Derwent abstract 1987-231555 discloses food for livestock containing 0.5-5 ppm of methyl-5-(phenylthio)-2-benzimidazole, which is supplied at low dosage.

WO 99/17625 describes a device for processing fodder.

US 5952 033 A describes gelatinized cereal product for use as pet food.

There is accordingly a need in the art for improved feeds including daily ration extrusion-processed feeds and methods of providing actives to animals in a manner which will avoid problems inherent in periodic dosing, while maintaining substantially constant therapeutic levels of actives such as ivermectin in the bloodstreams of the animals consuming the feeds on a daily basis.

### SUMMARY OF THE INVENTION

The present invention provides an animal feed comprising respective quantities of protein, fat, and starch, said animal feed having up to 1500 µg/kg feed of at least one active substantially uniformly dispersed therein, characterized in that the animal feed is a daily ration feed and is produced by:
   formulating a liquid containing said active;
   preconditioning a starting mixture containing said quantities of protein, fat, and starch by adding moisture to the starting mixture, subjecting the starting mixture to a temperature of from 20-98 °C for a period of from 15-600 seconds so that said mixture leaving the preconditioner has a moisture content of from 10-60% by weight, and adding said active-containing liquid to said mixture; and
   thereafter passing said preconditioned material into the elongated barrel of an extruder and subjecting the preconditioned material to maximum temperature conditions of from 20-175 °C and a maximum pressure condition of from 6.90 x 10⁵ Pa to 206.84 x 10⁵ Pa (100-3,000 psi), for a residence time of from 3-180 seconds,
   said extruded animal feed being said daily ration feed having uniform amounts of said active therein, and effective for continuously maintaining in the bloodstream of the animal consuming the feed on a daily basis a therapeutic amount of said active,
   said active being selected from the group consisting of antibiotics, steroids, anti-inflammatory agents, endoctocides, ectoparasiticides and mixtures thereof.

The present invention overcomes the problems outlined above and provides improved active containing daily ration feed products for animals such as cats, dogs, birds, exotics, horses, sheep, cattle, reptiles, other companion animals, and zoo animals and methods of preparing and using such feeds. The feeds are produced by extrusion and contain respective quantities of protein, fat and starch, together with a relatively minor amount of a desired active or drug. It is preferred that the potency of the active content of the feeds be maintained for at least six months at ambient temperature storage, more preferably nine months, and most preferably from about nine to twenty-four months at ambient temperature storage.

As noted previously, a large number of actives can be used in the context of the invention, so long as the actives can withstand feed processing conditions and retain their potency. The actives are antibiotics, steroids, anti-inflammatory agents, endectocides (e.g., dewormers such as heartworm-preventative drugs) and ectoparasiticides (e.g., drugs effective against fleas and ticks).

Through use of the feed products of the invention, an animal consuming the feeds on a daily basis receives a maintenance quantity of the active, so that the therapeutic effects thereof are realized. Normally, the active should be present in the extruded feeds at a level of at least 0.1 µg/kg of feed product more preferably from 2-1500 µg/kg of feed product, and most preferably from 4-1000 µg/kg of feed product, although specific active amounts may vary depending upon the particular active chosen. For example, ivermectin may be present at a level up to 1000 µg/kg of feed product on a dry basis (db), more preferably from 0.1-450 µg/kg of feed product (db), still more preferably from 4-250 µg/kg of feed product (db), and most preferably from 5-175 µg/kg of feed product (db). In other types of products within the ambit of the invention, the active may be present at a level of up to 0.75% by weight, more preferably up to 0.5% by weight, and still more preferably up to 0.1% by weight. Preferably, the active content of the feeds should be maintained for a period of at least about six months, more preferably at least about nine months, and most preferably from about nine to twenty-four months, at ambient temperature storage conditions. Alternately, the amount of active present in the feeds should be sufficient to administer from 0.025 to 5.35 µg active per kg of animal body weight per day, more preferably from 0.050 to 3.0 µg active per kg of animal body weight per day, and most preferably from 0.06 to 2.1 µg active per kg of animal body weight per day (assuming that each animal consumes 0.16 kg of the active-supplemented feed per kg of animal body weight per day).

As noted, a wide variety of extruded feeds can be used in the context of the invention. For example, typical dry extruded product having a moisture content of less than 10% by weight can be produced with added active. Similarly, semi-moist feeds having a moisture content on the order of 15-30% by weight are also suitable. In extruded feeds of these types, it is preferred that the active content be substantially uniformly dispersed throughout the feed. Alternately, pillow-type feeds can be produced having a soft, flowable matrix center surrounded by a shell of self-sustaining feed material; in such a case, the active content may be present only in the soft center matrix. In most cases, the extruded feed products of the invention should contain from 5-15% by weight moisture (wet basis), 15-30% by weight protein, more preferably from 18-25% by weight protein; from 3-24% by weight fat, more preferably from 5-20% by weight fat; and from 5-80% by weight starch, more preferably from 20-50% by weight starch. Generally, the extruded feeds should have a bulk density of from 30-700 g/l, more preferably from 140-400 g/l, and a water activity of from 0.1-0.99, more preferably from 0.6-0.75.

An important goal of the invention is to provide active-containing daily ration feeds which when consumed on a daily basis by animals will establish and maintain a therapeutic amount of active in the bloodstreams of the animals. In this way, the need for periodic dosing with relatively large amounts of active(s) is completely avoided, yet the beneficial effects of the active remain. To this end, the feeds should have sufficient active therein so that, when an animal consumes the feed at a rate of from 10-40 g of the feed per kg of the consuming animal's weight, the desired therapeutic amount of active is achieved.

During extrusion processing in accordance with the invention, starting farinaceous feed ingredients are fed into the elongated barrel of an extruder including at least one elongated, axially rotatable, helically flighted screw with an endmost extrusion die. During passage through the extruder barrel, the ingredients are subjected to elevated temperature, agitation and shear in order to cook the product. In preferred forms of the invention, the starting ingredients are first preconditioned prior to passage into the extruder barrel. Generally, during preconditioning the starting mixture is subjected to a temperature of from 20-98°C (more preferably from 90-97 ° C) for a period of from 15-600 seconds (more preferably from 170-190 seconds). The purpose of preconditioning is to initially moisturize and partially cook the starting material prior to entrance thereof into the extruder barrel. Advantageously, the material leaving the preconditioner has a moisture content of from 10-60% by weight, and more preferably from 21-23% by weight.

In the extruder, the preconditioned starting material is subjected to conditions of elevated heat, pressure and shear. Normally, the temperature conditions in the barrel are such as to achieve a maximum temperature of from 20°-175°C, and more preferably from about 65-120°F. Normal maximum pressure conditions are from 100- 3000 psi, and more preferably from 150-500 psi. Residence times in the extruder barrel usually range from 3-180 seconds, and more preferably from 20-40 seconds.

The active content of the extruded feeds can be added at a variety of locations during the process. One preferred technique is to prepare a dilute active solution which can be pumped at a known rate into the farinaceous ingredients during processing. For example, the active liquid may be added at the preconditioner, preferably adjacent the outlet thereof. Given the relatively small quantities of active employed in the feeds, it is generally important that there be sufficient time in the process to adequately mix in the active substantially uniformly throughout the other ingredients.

### DETAILED DESCRIPTION OF THE PREFERRED EMBODIMENTS

The following examples set forth presently preferred methods for the production of active-containing animal foods and related information. It is to be understood, however, that these examples are provided by way of illustration and nothing therein should be taken as a limitation upon the overall scope of the invention.

### Example 1

In this example, an ivermectin-containing dog food product was produced using a co-extrusion process. The dry farinaceous ingredients used in this example were (all percentages on a weight basis): wheat flour-14%; rice flour-15%; corn flour-32%; corn gluten meal-12%; poultry meal-8%; brewer's yeast-2%; sodium bicarbonate-0.6%; Thoxyquin-0.1%; potassium sorbate-0.3%; and sugar-5%. The liquid co-extruded mixture contained (all percentages on a weight basis: poultry fat-81.13%; GP (Glutamine Peptide)-11.32%; cheese powder-3.77%; and poultry meal-3.77%.

The extrusion equipment included a Wenger X-85 single screw extruder with a Wenger Model 7 DDC preconditioner. The extruder barrel was made up of a series of interconnected heads. The screw configuration, dies, adaptor parts, preconditioner shafts and beater elements were all Wenger equipment.

In order to effect the desired co-extrusion, a delivery pipe having approximately a 3/8" delivery nipple was inserted into the center of the die so that the liquid portion was directed through the die with a surrounding annulus of the extruded farinaceous mixture. The liquid portion was pumped through the delivery pipe at a rate which was approximately 30% of the extrusion rate of the farinaceous mixture. At the outlet of the extruder die, the product was cut using an knife and respective samples of the cut product were manually crimped using a hand-crimping tool. In this fashion, "pill ows" of the pet food were obtained, with an outer farinaceous ingredient shell and an inner flowable filling containing ivermectin.

Following this treatment, the product was dried to a moisture level of less than 10% by weight. Three samples from the dryer were subsequently frozen and another sample was placed in a plastic bag and stored at room temperature, for a period in excess of six months.

The following table sets forth the illustrative preconditioning and extrusion information.

**Table 1**

| DRY RECIPE INFORMATION | | |
|---|---|---|
| Dry Recipe Rate | kg/hr | 93 |
| Feed Screw Speed | rpm | 11 |

| PRECONDITIONING INFORMATION | | |
|---|---|---|
| Preconditioner Speed | rpm | 485 |
| Steam Flow to Preconditioner | kg/hr | 8 |
| Water Flow to Preconditioner | kg/hr | 21 |
| Preconditioner Discharge Temp. | °C | 66 |

| EXTRUSION INFORMATION | | |
|---|---|---|
| Extruder Shaft Speed | rpm | 516 |
| Extruder Motor Load | % | 75 |
| Control/Temperature 2nd Head | °C | 40 |
| Control/Temperature 3rd Head | °C | 51 |
| Control/Temperature 4th Head | °C | 39 |
| Control/Temperature 5th Head | °C | 48 |
| Control/Temperature 7th Head | °C | 45 |

| FINAL PRODUCT INFORMATION | | |
|---|---|---|
| Extruder Discharge Density | kg/m³ | 350 |

The products resulting from this test were analyzed to determine the content of ivermectin in the samples. In this analysis, each feed sample was ground in a Retsch mill at low speed using a 2 mm grating screen, so that the ground material would pass through a #10 mesh screen. A total of six samples, three frozen and three stored at room temperature, were processed. In each case, three 37.5 g of a sample was placed in a 250 ml bottle and 100 ml of methanol was added. The bottle was capped, the sample was sonicated for 20 minutes and shaken for 1 hour. 40 ml of the extract was added to a centrifuge tube and centrifuged for 5 minutes at 2000 rpm. 20 ml of the supernatant solution was then passed through a alumina column. The first five ml was rejected and the remainder of the liquid through the column was collected as a purified sample. 2 ml of the purified sample was mixed with a 5 ml mixture of acetonitrile:water (1:1), and a solid phase extraction (SPE) was performed in accordance with the procedure described in Doherty et al., Analytical Chemists International, 81:869(4) (1998). 2 ml of the working, 1% ivermectin sample standard was also nm through the SPE procedure to determine if any loss of ivermectin was taking place.

All samples from the SPE treatment were evaporated under nitrogen using an analytical evaporator with a water bath temperature of 50°C. The dried samples were reconstituted in 2 ml of HPLC mobile phase for analysis. Two samples were also prepared using 2 ml of the working standard ivermectin solution (containing 0.42 µg/ml) and were run before and after the feed samples.

The HPLC setup consisted of the following:

| | |
|---|---|
| Gilson 712 HPLC System Controller | |
| Gilson 305 pump, 231 sample injector, 401 dilutor and 115 UV detector | |
| Jones Chromatography column heater set at 30°C | |
| HPLC Analytical colum | Symmetry C₁₈, 5µ, 4.6 x 350 mm |
| Mobile Phase | Acetonitrile/methanol/water 53/35/7 |
| Flow rate | 1 mL/minute |
| UV Detection | 245 nm |

The results of the HPLC analyses (two injections of each feed sample and two injections of the working standard solution) confirmed that the pet food samples contained very close to the expected content (0.42 µg/kg) ofivermectin. In particular, the average ivermectin content of the three frozen and the ambient-stored samples was 0.43 µg/kg. This demonstrated that storage conditions (frozen versus ambient) had little effect upon ivermectin potency, and an excellent ivermectin stability.

### Example 2

In this example, an ivermectin-containing dog food was prepared using a Wenger TX-85 twin screw extruder equipped with a Model 16 Wenger DDC preconditioner. The dry ingredients fed to the extruder included (all percentages by weight basis): wheat middlings-18%; meat and bone meal-18%; soybean meal-18%; and corn-46%. In this run, two liquid dispersions were used which contained (all percentages by weight basis): first mixture, propylene glycol-11 lbs and water-11 lbs; second mixture, propylene glycol-48.82%; water-48.82%; Red No. 40 dye-1.86%; and ivermectin solution-0.50%. The amount of ivermectin used was calculated to provide a dose of approximately 1121.1 µg of ivermectin per kg of the dog food on a dry basis.

The extruder barrel was made up of interconnected heads. The rotating elements within the barrel included extruder shafts and other elements. The extruder was equipped with dies and adaptors, inserts, and a cutting knife with knife blades was used. The foregoing components as well as the preconditioners shafts and beater elements were all Wenger equipment.

In the process, the dry ingredients were fed to the preconditioner where steam and water was added to moisturize and partially precook the mixture. This preconditioned material was then fed to the inlet of the extruder in the usual fashion. The first liquid mixture was added to the outlet end of the preconditioner for passage into the extruder barrel along with the preconditioned material, over a period of about 11 minutes. Thereafter, the colored, ivermectin-containing liquid mixture was added over a period of about 22 minutes. Finally, additional quantities of the first water/propylene glycol liquid mixture was again added, over about 11 minutes. After extrusion, the product was dried in a Wenger dryer operating at 115°C, with two drying passes of 7 and 8.9 minutes respectively, followed by a cooler pass with 4.5 minutes retention time. The dryer discharge moisture was 6.25%, wb.

Samples were collected of the colored ivermectin-containing dispersion, the raw material mixture, preconditioned material leaving the preconditioner and extruded samples.

The following table sets forth illustrative preconditioning and extrusion conditions.

**Table 2**

| DRY RECIPE INFORMATION | | |
|---|---|---|
| Dry Recipe Moisture | % w b | 9.56 |
| Dry Recipe Density | kg/m³ | 570 |
| Dry Recipe Rate | kg/hr | 2618 |
| Feed Screw Speed | rpm | 205 |

| PRECONDITIONING INFORMATION | | |
|---|---|---|
| Preconditioner Speed | rpm | 250 |
| Steam Flow to Preconditioner | kg/hr | 224 |
| Water Flow to Preconditioner | kg/hr | 362 |
| Preconditioner Additive 1 Rate | kg/hr | 57 |
| Preconditioner Discharge Temp. | °C | 90 |

| EXTRUSION INFORMATION | | |
|---|---|---|
| Extruder Shaft Speed | rpm | 700 |
| Extruder Motor Load | % | 67 |
| Steam Flow to Extruder | kg/hr | 84 |
| Water Flow to Extruder | kg/hr | 112 |
| Control/Temperature 1st Head | °C | 50/57 |
| Control/Temperature 2nd Head | °C | 50/86 |
| Control/Temperature 3rd Head | °C | 40/52 |
| Control/Temperature 4th Head | °C | 40/75 |
| Head/Pressure | kPa | 900 |
| Knife Drive Speed | rpm | 905 |

| FINAL PRODUCT INFORMATION | | |
|---|---|---|
| Extruder Discharge Density | kg/m³ | 368 |
| Extruder Performance | | Stable |

The dog food from this run was fed *ad libitum* to an intact female mixed breed dog weighing about 10 kg. On day 7, blood was drawn from the dog four hours after eating and stored in an anti-coagulant tube with calcium EDTA in a refrigerator. Seven days later, the same dog was again fed the ivermectin-containing feed *ad libitum* and blood was collected four hours post-feeding. This sample was also refrigerated in the same fashion as the first sample.

The blood samples were then analyzed to determine the content of ivermectin therein, using HPLC. The procedure used was described in Dickinson, Journal of Chromatography, 58:250-257 (1990). In this procedure, 0.5 ml of each blood sample was purified using solid phase extraction (SPE) cartridges and dissolved in a small volume of mobile phase for injection onto the HPLC column. The method has a limit of detection of about 2 ng/ml and uses an internal standard. After preparation of the internal standard, a standard curve is constructed using ivermectin-spiked blood samples. A known 1% ivermectin sample was used as the primary standard.

The blood samples from the dog were then analyzed for ivermectin content with HPLC peak heights corrected using the internal standard. The HPLC setup consisted of the following:

| | |
|---|---|
| Gilson 712 HPLC System Controller | |
| Gilson 305 pump, 231 sample injector, 401 dilutor and 115 UV detector | |
| Jones Chromatography column heater set at 56°C | |
| HPLC Analytical column: | Coulter-Beckman UltraSphere XL C₁₈, 3µ, 4.6 x 70 mm |
| Mobile Phase: | Acetonitrile/methanol/water 49/33/18 |
| Flow Rate: | 1 mL/minute |
| UV Detection: | 245 nm |

The results of this study demonstrated that the dog blood samples contained ivermectin in the range of about 5-8 ng/ml.

### EXAMPLE 3

In this example a series of extrusion runs were performed to determine the consistency ofmetering of ivermectin into a dog food mixture during extrusion. In each case, the farinaceous mixture included the following ingredients (all percentages on a weight basis): com- 35.93%; poultry meal- 28.94%; rice- 22.95%; corn gluten meal- 11.98%; vitamin premix- 0.10%; and mineral premix-0.10%. Three ivermectin-containing liquids were prepared, containing: Recipe #1, propylene glycol-8.60 pounds; water-8.60 pounds; red #40 dye-160 grams; ivermectin solution-0.212 ml; Recipe #2, propylene glycol-8.60 pounds; water-8.60 pounds; red #40 dye-160 grams; ivermectin solution- 0.433 ml; Recipe #3, propylene glycol-8.60 pounds; water-8.60 pounds; red #40 dye-160 grams; ivermectin solution-1.279 ml. In each run 8.0 kg of a respective ivermectin recipe was added to the farinaceous ingredients at the exit of the preconditioner, prior to entering the extruder barrel. The recipes were added at a rate equal to 2% of the farinaceous mixture rate. The target for the runs using Recipe #1 was 6 µg ivermectin/kg of feed; for runs using Recipe #2, 12 µg/kg; and for runs using Recipe #3, 36µg/kg.

The extruder system employed was a Wenger model TX 57 twin screw extruder with a model 2 DDC preconditioner. The extruder barrel was equipped with an extrusion die, a knife assembly was used to cut extrudate.

The following table sets forth the preconditioning and extrusion information collected during this series of runs. In runs 101-103, Recipe #1 was used; in runs 104-106, Recipe #2 was used; and in runs 107-109, Recipe #3 was used. As the extrudates emerged from the die, they were cut using the knife assembly and dried in a Wenger multiple-pass drier. Samples were collected at 15 minutes, 30 minutes and 45 minutes from the preconditioner, extruder and drier.

**Table 3**

| DRY RECIPE INFORMATION: | | | | | | | | | | |
|---|---|---|---|---|---|---|---|---|---|---|
| | | 101 | 102 | 103 | 104 | 105 | 106 | 107 | 108 | 109 |
| Dry Recipe Density | kg/m³ | 494 | 494 | 494 | 494 | 494 | 494 | 494 | 494 | 494 |
| Dry Recipe Rate | kg/hr | 400 | 400 | 400 | 390 | 392 | 390 | 387 | 397 | 392 |
| Feed Screw Rate | rpm | 48 | 53 | 55 | 49 | 52 | 52 | 56 | 54 | 54 |

| PRECONDITIONING INFORMATION: | | | | | | | | | | |
|---|---|---|---|---|---|---|---|---|---|---|
| Preconditioner Speed | rpm | 350 | 350 | 350 | 350 | 350 | 350 | 350 | 350 | 350 |
| Steam Flow to Preconditioner | kg/hr | 36 | 35.8 | 35.9 | 36.1 | 35.9 | 35.8 | 36 | 36.1 | 35.9 |
| Water Flow to Preconditioner | kg/hr | 48 | 48.1 | 48.3 | T47.7 | 47.9 | 48.1 | 48 | 48.2 | 48.1 |
| Preconditioner Additive 1 Rate | kg/hr | 8 | 7.9 | 8.05 | 7.8 | 7.95 | 7.84 | 8.12 | 8.03 | 8.02 |
| Preconditioner Discharge Temp. | °C | 86 | 85 | 85 | 86 | 86 | 86 | 85 | 85 | 85 |
| Moisture Entering Extruder | %w b | 16.26 | 17.04 | 19.14 | 18.96 | 16.47 | 18.18 | 16.14 | 18.97 | 18.98 |

| EXTRUSION INFORMATION: | | | | | | | | | | |
|---|---|---|---|---|---|---|---|---|---|---|
| Extruder Shaft Speed | rpm | 426 | 427 | 425 | 427 | 426 | 426 | 426 | 426 | 425 |
| Extruder Motor Load | % | 53 | 45 | 61 | 54 | 52 | 67 | 49 | 51 | 52 |
| Steam Flow to Extruder | kg/hr | 12 | 13.1 | 709 | 8 | 7.9 | 8 | 8.1 | 8 | 8 |

| Water Flow to Extruder | kg/hr | 24 | 24 | 24.1 | 24 | 24 | 23.8 | 24 | 24 | 23.9 |
|---|---|---|---|---|---|---|---|---|---|---|
| Control/Temp. 1st Head | °C | 40/52 | 40/52 | 40/52 | 40/53 | 40/55 | 40/52 | 40/53 | 40/55 | 40/54 |
| Control/Temp. 2nd Head | °C | 60/60 | 60/60 | 60/59 | 60/60 | 60/60 | 60/59 | 60/59 | 60/59 | 60/60 |
| Control/Temp. 3rd Head | °C | 80/79 | 80/80 | 80/81 | 80/80 | 80/80 | 80/81 | 80/80 | 80/80 | 80/79 |
| Control/Temp. 4th Head | °C | 60/67 | 60/67 | 60/67 | 60/65 | 60/65 | 60/66 | 60/65 | 60/65 | 60/64 |
| Head/Pressure | kPa | 1710 | 1600 | 1980 | 1660 | 1770 | 1910 | 1960 | 1980 | 1830 |
| Knife Drive Speed | rpm | 1324 | 1324 | 1325 | 1492 | 1443 | 1493 | 1493 | 1492 | 1491 |

| FINAL PRODUCT INFORMATION: | | | | | | | | | | |
|---|---|---|---|---|---|---|---|---|---|---|
| Extruder Discharge Moisture | % w b | 20.43 | 19.79 | 20.4 | 21.32 | 21.46 | 21.97 | 22.12 | 22.83 | 22.71 |
| Extruder Discharge Density | kg/m³ | 312 | 374 | 338 | 400 | 349 | 352 | 336 | 336 | 400 |
| Extruder Performance | | Stable | Stable | Stable | Stable | Stable | Stable | Stable | Stable | Stable |
| Dried Product Moisture | % w b | 2.75 | 2.12 | 4.67 | 9.38 | 9.74 | 10.18 | 7.45 | 9.4 | 8.0 |

The dried samples were analyzed to determine ivermectin content, using the technique described in Example 1. The results from the Recipe #1, #2 and #3 runs were averaged, with the following results. For the Recipe #1 runs (101-103), the ivermectin content was 6.02 µg/kg (db); for the Recipe #2 runs (104-106), the ivermectin content was 11.99 µg/kg (db); and for the Recipe #3 runs (107-109), the ivermectin content was 35.98 µg/kg (db). This confirms that the processing technique of this Example gives extremely close ivermectin contents, as compared with the pre-extrusion goals.

### Example 4

In another series of tests, the methods described in Example 3 were followed to produce feed products containing 0.1, 0.2, 2, and 20 µg ivermectin per kg of feed product (db). The dried samples were analyzed to determine ivermectin content, using the technique described in Example 1. The final products contained 0.1, 0.2, 2, and 20 µg ivermectin per kg of feed product. This confirms that the processing technique of this Example gives extremely close ivermectin contents, as compared with the pre-extrusion goals

### Example 5

In this example, a series of extrusion runs were carried out with dog food products containing different active ingredients. The equipment employed was a Wenger laboratory-scale X-5 extruder. The actives used in the respective runs were: Methoprene (insect growth regulator, Run 002); Lufenuron (insect growth regulator, chemically dissimilar to Methoprene, Run 003); Praziquantel (tapeworm treatment, Run 004); Enrofloxacin (potent broad spectrum antibiotic, Run 005); Dexamethasone (steroid of the cortisone type, Run 006); Ibuprofen (non-steroidal anti-inflammatory drug, Run 007); Fenbendazole (mammal dewormer, Run 008); Oxytetracycline (widely used antibiotic, Run 009); Ivermectin, Methoprene, Praziquantal cocktail (antiparasitical combination, Run 010); Imidaccopria (imidacloprid, Run 011); Amoxicillin (broad spectrum antibiotic, Run 012); Tribrissen (antibiotic, Run 013); Doramectin (broad spectrum dewormer and anthelmintic, Run 014).

In particular, the recipes for each run are set forth in the following table:

**Table 4**

| Recipe-Run 001 | By Weight |
|---|---|
| Corn | 35.9281% |
| Poultry Meal | 28.9421% |
| Rice | 22.9541% |
| Corn Gluten Meal | 11.9760% |
| Lasi Pet Premix | 0.0998% |
| Trace Mineral #95 | 0.0998% |
| Total | 100.0000% |

| | |
|---|---|
| *0.980 kg of water was added to the above mixture | |

| Recipe-Run 002 | By Weight |
|---|---|
| Corn | 35.7982% |
| Poultry Meal | 28.8374% |
| Rice | 22.8711% |
| Corn Gluten Meal | 11.9327% |
| Lasi Pet Premix | 0.0994% |
| Trace Mineral #95 | 0.0994% |
| Hartz Methoprene Capsule Content | 0.3618% |
| Total | 100.0000% |

| | |
|---|---|
| *0.980 kg of water was added to the above mixture Calculated Active content in batch = 0.0051 kg | |

| Recipe- Run 003 | By Weight |
|---|---|
| Corn | 35.7982% |
| Poultry Meal | 28.8374% |
| Rice | 22.8711% |
| Corn Gluten Meal | 11.9327% |
| Lasi Pet Premix | 0.0994% |
| Trace Mineral #95 | 0.0994% |
| Lufenuron- Novartis | 0.01571% |
| Total | 99.7954 % |

| | |
|---|---|
| *0.980 kg of water was added to the above mixture Calculated Active content in batch = 0.0053 kg | |

| Recipe- Run 004 | By Weight |
|---|---|
| Corn | 35.2250% |
| Poultry Meal | 28.3757% |
| Rice | 22.5049% |
| Corn Gluten Meal | 11.7417% |
| Lasi Pet Premix | 0.0978% |
| Trace Mineral #95 | 0.0978% |
| Bayer Droncit (Praziquantel) | 1.0176% |
| Propylene Glycol | 0.1571% |
| Total | 100.0000% |

| Recipe-Run 005 | By Weight |
|---|---|
| Corn | 35.5731% |
| Poultry Meal | 28.6561% |
| Rice | 22.7273% |
| Corn Gluten Meal | 11.8577% |
| Lasi Pet Premix | 0.0988% |
| Trace Mineral #95 | 0.0988% |
| Bayer Baytril Injectable (Enfloxacin) | 0.9881% |
| Total | 100.0000% |

| | |
|---|---|
| *0.880 kg of water was added to the above mixture Calculated Active content in batch = 0.0028 kg *0.930 kg of water was added to the above mixture Calculated Active content in batch = 0.0050 kg | |

| Recipe-Run 006 | By Weight |
|---|---|
| Corn | 35.6679% |
| Poultry Meal | 26.3158% |
| Rice | 20.8711% |
| Corn Gluten Meal | 10.8893% |
| Lasi Pet Premix | 0.0907% |
| Trace Mineral #95 | 0.0907% |
| Dexamethasone Solution | 9.0744% |
| Total | 100.0000% |

| | |
|---|---|
| *0.480 kg of water was added to the above mixture Calculated Active content in batch = 0.0010 kg | |

| Recipe-Run 007 | By Weight |
|---|---|
| Corn | 35.8728% |
| Poultry Meal | 28.8975% |
| Rice | 22.9187% |
| Corn Gluten Meal | 11.9576% |
| Lasi Pet Premix | 0.0996% |
| Trace Mineral #95 | 0.0996% |
| Ibuprofen | 0.1541% |
| Total | 100.0000% |

| | |
|---|---|
| *0.980 kg of water was added to the above mixture Calculated Active content in batch = 0.0050 kg | |

| Recipe-Run 008 | By Weight |
|---|---|
| Corn | 35.5554% |
| Poultry Meal | 25.6419% |
| Rice | 22.7160% |
| Corn Gluten Meal | 11.8518% |
| Lasi Pet Premix | 0.0988% |
| Trace Mineral #95 | 0.0988% |
| Pavacur- Febendzole Paste | 01.0374% |
| Total | 100.0000% |

| | |
|---|---|
| *0.980 kg of water was added to the above mixture Calculated Active content in batch = 0.005052 kg | |

| Recipe-Run 009 | By Weight |
|---|---|
| Corn | 35.5731% |
| Poultry Meal | 28.6561% |
| Rice | 22.7273% |
| Corn Gluten Meal | 11.8577% |
| Lasi Pet Premix | 0.0988% |
| Trace Mineral #95 | 0.0988% |
| Maxim 200- Oxytetracycline Solution | 0.9881% |
| Total | 100.0000% |

| | |
|---|---|
| *0.930 kg of water was added to the above mixture Calculated Active content in batch = 0.0050 kg | |

| Recipe-Run 010 | By Weight |
|---|---|
| Corn | 35.4801% |
| Poultry Meal | 28.5812% |
| Rice | 22.6678% |
| Corn Gluten Meal | 11.8267% |
| Lasi Pet Premix | 0.0986% |
| Trace Mineral #95 | 0.0986% |
| Equvalan Paste- Ivermectin | 0.1344% |
| Hartz Methoprene Capsule Content | 0.1271% |
| Bayer Droncit (Praziquantel) | 0.9856% |
| Total | 100.0000% |

| | |
|---|---|
| *0.980 kg of water was added to the above mixture Calculated Ivermectin Active content in batch = 0.000114 kg Calculated Methoprene Active content in batch = 0.005082 kg Calculated Praziquantel Active content in batch = 0.00284 kg | |

| Recipe-Run 011 | By Weight |
|---|---|
| Corn | 35.7001% |
| Poultry Meal | 28.7584% |
| Rice | 22.8084% |
| Corn Gluten Meal | 11.9000% |
| Lasi Pet Premix | 0.0992% |
| Trace Mineral #95 | 0.0992% |
| Bayer Advantage-Imidacloprid | 0.6347% |
| Total | 100.0000% |

| | |
|---|---|
| *0.948 kg of water was added to the above mixture Calculated Active content in batch = 0.002912 kg | |

| Recipe-Run 012 | By Weight |
|---|---|
| Corn | 35.8905% |
| Poultry Meal | 28.9118% |
| Rice | 22.9300% |
| Corn Gluten Meal | 11.9635% |
| Lasi Pet Premix | 0.0997% |
| Trace Mineral #95 | 0.0997% |
| Amoxicillin-Antibiotic | 0.1049% |
| Total | 100.0000% |

| Recipe-Run 013 | By Weight |
|---|---|
| Corn | 35.8802% |
| Poultry Meal | 28.9035% |
| Rice | 22.9234% |
| Corn Gluten Meal | 11.9601 % |
| Lasi Pet Premix | 0.0997% |
| Trace Mineral #95 | 0.0997% |
| Tribrissen- Antibiotic | 0.1336% |
| Total | 100.0000% |

| | |
|---|---|
| *0.980 kg of water was added to the above mixture Calculated Active content in batch = 0.005 kg *0.980 kg of water was added to the above mixture Calculated Active content in batch = 0.00576 kg | |

| Recipe-Run 014 | By Weight |
|---|---|
| Corn | 35.8566% |
| Poultry Meal | 28.8845% |
| Rice | 22.9084% |
| Corn Gluten Meal | 11.9522% |
| Lasi Pet Premix | 0.0996% |
| Trace Mineral #95 | 0.0996% |
| Doramectin (Dectomax) | 0.1992 |
| Total | 100.0000% |

| | |
|---|---|
| *0.970 kg of water was added to the above mixture Calculated Active content in batch = 0.0001 kg | |

The X-5 extruder included seven interconnected heads with a single extruder shaft supporting rotating elements. The X-5 was also equipped with a Wenger die/adaptor. The extrudates were manually cut upon emerging from the die and were dried in a laboratory drier to a moisture content less than 10% by weight.

In each run the active ingredient(s) were diluted into a miscible liquid (water orpropylene glycol) and combined with 0.5 kg of the recipe to make a premix. This premix was then loaded into a Hobart mixer along with the remaining contents of the batch (total of 5 kg in each case) and mixed to obtain the final recipe for extrusion. The individual batches were loaded into the feeding bin and the extrusion runs were started. Samples were taken at regular intervals of approximately 5 minutes after stable conditions were achieved. Some samples were taken "as is" from the extruder without drying and were frozen. Other dried samples were bagged and maintained at ambient temperature.

## Claims

1. An animal feed comprising respective quantities of protein, fat, and starch, said animal feed having up to 1500 µg/kg feed of at least one active substantially uniformly dispersed therein, **characterized in that** the animal feed is a daily ration feed and is produced by:
formulating a liquid containing said active;
preconditioning a starting mixture containing said quantities of protein, fat, and starch by adding moisture to the starting mixture, subjecting the starting mixture to a temperature of from 20-98 °C for a period of from 15-600 seconds so that said mixture leaving the preconditioner has a moisture content of from 10-60% by weight, and adding said active-containing liquid to said mixture; and
thereafter passing said preconditioned material into the elongated barrel of an extruder and subjecting the preconditioned material to maximum temperature conditions of from 20-175 °C and a maximum pressure condition of from 6.90 x 10⁵ Pa to 206.84 x 10⁵ Pa (100-3,000 psi), for a residence time of from 3-180 seconds,
said extruded animal feed being said daily ration feed having uniform amounts of said active therein, and effective for continuously maintaining in the bloodstream of the animal consuming the feed on a daily basis a therapeutic amount of said active,
said active being selected from the group consisting of antibiotics, steroids, anti-inflammatory agents, endoctocides, ectoparasiticides and mixtures thereof.

2. The feed of claim 1, said active being present in the extruded product feed at a level of at least 2 µg active/kg of the extruded product feed.

3. The feed of claim 2, said level being from 2-1500 µg active/kg feed.

4. The feed of claim 1, said extruded feed product selected from the group consisting of dry and semi-moist extruded feed products.

5. The feed of claim 1, said active being present at a level so that, when an animal consumes the feed at a daily rate of from 10-40 g feed per kg of the animal's weight, a therapeutically effective amount of the active is established and maintained in the animal's bloodstream.

6. The feed of claim 1, wherein the feed is produced by adding said active containing liquid formulation into the preconditioner.

## Patentansprüche

1. Tierfutter, das jeweilige Mengen an Protein, Fett und Stärke umfasst, wobei das Tierfutter bis zu 1500 µg/kg Futter von mindestens einem darin im Wesentlichen gleichförmig dispergierten Wirkstoff enthält, **dadurch gekennzeichnet, dass** das Tierfutter eine Tagesration an Futter ist und hergestellt wird, indem
eine Flüssigkeit formuliert wird, die den Wirkstoff enthält,
eine Ausgangsmischung, die die Mengen an Protein, Fett und Stärke enthält, vorkonditioniert wird indem Feuchtigkeit zu der Ausgangsmischung zugesetzt wird, die Ausgangsmischung 15 bis 600 Sekunden lang einer Temperatur von 20 bis 98°C unterzogen wird, so dass die Mischung, die den Vorkonditionierer verlässt, einen Feuchtigkeitsgehalt von 10 bis 60 Gew.-% aufweist, und die Wirkstoff enthaltende Flüssigkeit zu der Mischung gegeben wird, und
anschließend das vorkonditionierte Material in die längliche Trommel eines Extruders geführt wird und das vorkonditionierte Material maximalen Temperaturbedingungen von 20 bis 175°C und einer maximalen Druckbedingung von 6,90 x 10⁵ Pa bis 206,84 x 10⁵ Pa (100 bis 3000 psi) während einer Verweilzeit von 3 bis 180 Sekunden unterzogen wird,
wobei das extrudierte Tierfutter die tägliche Futterration mit gleichförmigen Mengen des Wirkstoffs darin ist und die kontinuierliche Aufrechterhaltung einer therapeutischen Menge des Wirkstoffs im Blutstrom des Tieres bewirkt, dass das Futter auf täglicher Basis konsumiert,
wobei der Wirkstoff aus der Gruppe bestehend aus Antibiotika, Steroiden, entzündungshemmenden Mitteln, Endectociden, Ectoparasiticiden und Mischungen derselben ausgewählt ist.

2. Futter nach Anspruch 1, bei dem der Wirkstoff in dem extrudierten Futter in einer Menge von mindestens 2 µg Wirkstoff / kg des extrudierten Futterprodukts vorhanden ist.

3. Futter nach Anspruch 2, bei dem die Menge 2 bis 1500 µg Wirkstoff / kg Futter beträgt.

4. Futter nach Anspruch 1, bei dem das extrudierte Futterprodukt aus der Gruppe bestehend aus trockenen und halbfeuchten extrudierten Futterprodukten ausgewählt ist.

5. Futter nach Anspruch 1, bei dem der Wirkstoff in einer solchen Menge vorhanden ist, dass, wenn ein Tier das Futter mit einer täglichen Rate von 10 bis 40 g Futter pro Kilogramm des Tiergewichts konsumiert, eine therapeutisch wirksame Menge des Wirkstoffs in dem Tierblutstrom aufge-baut und aufrechterhalten wird.

6. Futter nach Anspruch 1, bei dem das Futter hergestellt wird, indem die Wirkstoff enthaltende flüssige Formulierung in den Vorkonditionierer gegeben wird.

## Revendications

1. Aliment pour animaux comprenant des quantités respectives de protéine, matière grasse et d'amidon, ledit aliment pour animaux présentant jusqu'à 1500 µg/kg d'aliment d'au moins un ingrédient actif dispersé de manière sensiblement uniforme dans celui-ci, **caractérisé en ce que** l'aliment pour animaux est une ration alimentaire journalière et est produit par :
la formulation d'un liquide contenant ledit ingrédient actif ;
le préconditionnement d'un mélange de départ contenant lesdites quantités de protéine, matière grasse et amidon en ajoutant de l'eau au mélange de départ, en soumettant le mélange de départ à une température allant de 20 à 98 °C pendant une période de 15 à 600 secondes de sorte que ledit mélange quittant l'appareil de préconditionnement présente une teneur en eau allant de 10 à 60 % en poids, et en ajoutant ledit liquide contenant l'ingrédient actif audit mélange ; et
ensuite le passage de ladite matière préconditionnée dans la cuve allongée d'une extrudeuse et la soumission de la matière préconditionnée à des conditions de température maximale de 20 à 175 °C et une condition de pression maximale de 6,90 x 10⁵ Pa à 206,84 x 10⁵ Pa (100 à 3000 psi) pendant un temps de séjour de 3 à 180 secondes,
ledit aliment pour animaux extrudé étant ladite ration alimentaire journalière présentant des quantités uniformes dudit ingrédient actif dans celle-ci, et étant efficace pour le maintien continu dans le système sanguin de l'animal consommant l'aliment quotidiennement d'une quantité thérapeutique dudit ingrédient actif,
ledit ingrédient actif étant choisi parmi le groupe comprenant les antibiotiques, les stéroïdes, les agents anti-inflammatoires, les endoctocides, les ectoparasiticides et leurs mélanges.

2. Aliment selon la revendication 1, ledit ingrédient actif étant présent dans le produit alimentaire extrudé à un taux d'au moins 2 µg d'ingrédient actif/kg du produit alimentaire extrudé.

3. Aliment selon la revendication 2, ledit taux allant de 2 à 1500 µg d'ingrédient actif/kg d'aliment.

4. Aliment selon la revendication 1, ledit produit alimentaire extrudé étant choisi parmi le groupe comprenant les produits alimentaires extrudés secs et semi-humides.

5. Aliment selon la revendication 1, ledit ingrédient actif étant présent à un taux tel que, lorsqu'un animal consomme l'aliment à un taux journalier de 10 à 40 g d'aliment par kg du poids de l'animal, une quantité thérapeutiquement efficace de l'ingrédient actif est établie et maintenue dans le système sanguin de l'animal.

6. Aliment selon la revendication 1, dans lequel l'aliment est produit par ajout de ladite formulation liquide contenant l'ingrédient actif dans l'appareil de préconditionnement.
